# DEMANDE DE BREVET EUROPEEN

(11) **EP 0 748 614 A1**
(43) Date de publication de la demande: **18.12.1996**
(21) Numéro de dépôt: 96401265.2
(22) Date de dépôt: 11.06.1996
(51) Int. Cl.: A61B 17/28, A61B 17/58, B26B 17/02

(54) **Dispositif développeur d'efforts d'une pince coupante**

(30) Priorité: 15.06.1995 FR 9507157
(71) Demandeur: EIS INSTRUMENTS, 52000 Chaumont (FR)
(72) Inventeur: Lanzoni, Maurice, 52000 Chaumont (FR)
(74) Mandataire: Debay, Yves

(57) **Abrégé**

Ce dispositif développeur d'efforts d'une pince du type munie de deux becs (2, 3) et de deux poignées (4, 5) comporte :
- une mâchoire (6A) ;
- un système à biellettes (6B) articulé avec la mâchoire (6A) ;
- un piston (7) relié par son extrémité avant au système à biellettes (6B) et logé à coulissement à l'intérieur d'une première poignée (4) ;
- des moyens complémentaires (9) d'entraînement du piston (7) et des moyens anti-retour agissant alternativement sur celui-ci de manière à provoquer, par mouvements successifs de rapprochement et d'éloignement de la seconde poignée (5) par rapport à la première poignée (4), un déplacement du piston (7) vers l'avant et à déformer la mâchoire (6A) par l'intermédiaire du système à biellettes (6B) pour rapprocher les deux becs (2, 3) l'un vers l'autre.

Application aux pinces chirurgicales.

## Description

La présente invention est relative à un dispositif développeur d'efforts d'une pince du type munie de deux becs et de deux poignées. Elle s'applique en particulier aux pinces chirurgicales coupantes.

Lors d'opérations chirurgicales, par exemple d'ostéosynthèse rachidienne ou d'enclouages centraux médulaires élastiques, il est courant d'employer des plaques ou des tiges métalliques pour réaliser des montages d'ostéosynthèse ou pour la fixation de fragments osseux résultant d'une fracture.

Dans le cas d'interventions sur le rachis avec des tiges ou des plaques, diverses situations peuvent se présenter.

En cours de préparation d'ajustement des plaques ou des tiges aux dimensions de l'anatomie du patient, le chirurgien doit pouvoir couper celles-ci à la bonne longueur, en tenant l'implant d'une main et en coupant avec l'autre main les plaques ou les tiges avec une pince, tout en développant un effort minimum.

Par ailleurs, en fin d'intervention, lorsque les réductions sont faites et les fixations assurées, certaines parties aux extrémités des montages d'ostéosynthèse deviennent proéminentes et donc indésirables. Ces extrémités doivent également être éliminées par cisaillage in situ.

En cas d'ablation de ces montages d'ostéosynthèse devenus indésirables après consolidation, il arrive que le retrait des tiges ou des plaques ne puisse pas se faire par simple ouverture des fixations reliant ces tiges entre elles. Il est donc indispensable de cisailler les tiges ou les plaques de part et d'autre des crochets et des vis pédiculaires afin de pouvoir extraire ceux-ci sans être géné par les longues tiges.

Dans divers cas, il est également nécessaire de pouvoir travailler tout en restant dans l'espace alloué par l'anatomie qui est très réduit. Il faut donc pouvoir apporter l'énergie nécessaire à la coupe des implants.

Dans le cas de pose ou d'ablation de tiges métalliques ou de clous centraux médulaires, les entailles d'accès sont réduites au minimum indispensable afin de favoriser une cicatrisation ultérieure rapide et discrète.

Dans de telles ouvertures petites et peu profondes, il faut soit aller couper les bouts des tiges surabondants, soit aller agripper l'extrémité rémanante en cas d'ablation par extraction.

D'autre part, dans le cas du cisaillage, les becs des pinces doivent être peu encombrants et doivent résister à l'effort de coupe.

Dans le cas de l'extraction des tiges, l'arrimage de la partie disponible de la tige doit se faire par un pincement extrêmement puissant pour ne pas riper sous l'effort d'extraction.

D'une manière générale, les becs et les pinces utilisées pour ce travail doivent développer une puissance de pincement importante, tout en étant très peu encombrants pour intervenir in situ, dans des espaces alloués par l'anatomie très réduits.

Or, les pinces habituellement utilisées possèdent une puissance de coupe limitée ne permettant pas de couper des tiges de diamètre sensiblement voisin de 7 mm. Dans le cas contraire, l'encombrement de leurs becs est tel que tout travail in situ est prohibé, et de plus la manipulation de telles pinces est laborieuse, pouvant même devenir dangereuse. En effet, les efforts nécessaires pour obtenir le sectionnement des tiges demandent l'action de deux personnes simultanément sur ces pinces, dont la longueur des manches dépassent 0,5 m, ce qui peut engendrer, de la part du chirurgien, la perte de la maîtrise du geste opératoire.

Par ailleurs, les pinces connues sont adaptées pour un besoin chirurgical spécifique : elles ne peuvent pas être à la fois des pinces de coupe et des pinces de serrage.

L'invention a pour but de fournir une pince chirurgicale qui remédie aux inconvénients précités, c'est-à-dire qui soit modulaire et d'un encombrement réduit tout en développant une puissance importante, et manipulable d'une seule main, et ce par des moyens simples, efficaces et peu coûteux.

A cet effet, l'invention a pour objet un dispositif développeur d'efforts d'une pince du type munie de deux becs et de deux poignées, caractérisé en ce qu'il comporte :
- une mâchoire formée de deux biellettes coudées dans des sens opposés et articulées, au niveau de leur partie coudée, indépendamment l'une de l'autre, les deux becs étant positionnés à une de leurs extrémités ;
- un système à biellettes articulé avec les deux biellettes de la mâchoire, l'ensemble mâchoire et système à biellettes ayant sensiblement la forme d'un quadrilatère déformable ;
- un piston relié par son extrémité avant au système à biellettes au niveau d'un sommet de ce système opposé au sommet de la mâchoire portant les becs, le piston étant logé à l'intérieur d'une première poignée et guidé à coulissement par celle-ci ;
- des moyens de maintien de la seconde poignée par rapport à la première poignée solidaires du piston et prenant appui sur la première poignée;
- des moyens complémentaires d'entraînement mécanique du piston à l'intérieur de la première poignée, portés par chacune des deux poignées; et
- des moyens anti-retour du piston portés par la première poignée ;
les moyens complémentaires d'entraînement du piston et les moyens anti-retour agissant alternativement sur celui-ci de manière à provoquer, par mouvements successifs de rapprochement et d'éloignement de la seconde poignée par rapport à la première poignée, un déplacement du piston vers l'avant et à déformer la mâchoire par l'intermédiaire du système à biellettes pour rapprocher les deux becs l'un vers l'autre.

Le dispositif développeur selon l'invention peut comporter une ou plusieurs des caractéristiques suivantes :
- les moyens d'entraînement mécanique comprennent une première crémaillère portée fixement par la première poignée et une deuxième crémaillère, de longueur inférieure à la première crémaillère, articulée sur la deuxième poignée et placée en regard de la première crémaillère, de manière à s'engréner mutuellement lors du rapprochement des deux poignées ;
- les moyens d'entraînement mécanique comprennent une crémaillère portée fixement par la première poignée et un cliquet porté par la deuxième poignée de manière à coopérer avec ladite crémaillère lors du rapprochement des deux poignées ;
- le dispositif comporte des moyens élastiques portés par les deux poignées, de façon à solliciter ces poignées en éloignement l'une de l'autre et à appliquer la deuxième crémaillère ou le cliquet sur la première crémaillère lors du rapprochement des poignées ;
- le sytème à biellettes comprend deux paires de biellettes, chaque paire étant articulée à l'extrémité avant du piston et à l'une des deux biellettes de la mâchoire ;
- les moyens anti-retour du piston comprennent une gâchette portée par la première poignée et coopérant avec une crémaillère supplémentaire placée sur le piston ;
- les moyens de maintien de la seconde poignée par rapport à la première poignée comprennent un étrier orienté à l'opposé de la première crémaillère et dont les deux joues sont parallèles et disposées de part et d'autre de la première poignée ;
- les extrémités arrière des joues sont solidaires du piston et la deuxième poignée est articulée par deux pivots placés à l'avant des joues ;
- l'étrier est en appui sur la première poignée par l'intermédiaire d'un galet sur la face de ladite première poignée opposée à celle portant la première crémaillère ;
- les moyens anti-retour du piston sont désenclenchables ;
- le dispositif comporte des moyens élastiques sollicitant le piston vers l'arrière lorsque les moyens anti-retour sont désenclenchés ; et
- les becs sont des becs amovibles.

L'invention a en outre pour objet une pince coupante comprenant le dispositif défini tel que ci-dessus, et dans laquelle les becs sont soit des becs de cisaillement, soit des becs de serrage.

Un exemple de réalisation va maintenant être décrit en regard des dessins annexés, sur lesquels :
- la figure 1 est une vue en perspective d'une pince et de son dispositif développeur d'efforts selon l'invention ;
- la figure 2 est une coupe longitudinale du dispositif développeur d'efforts de la figure 1, en position de repos ; et
- les figures 3 à 5 sont des coupes longitudinales du dispositif développeur d'efforts de la figure 1, représenté dans les états successifs conduisant à la fermeture des becs de la pince.

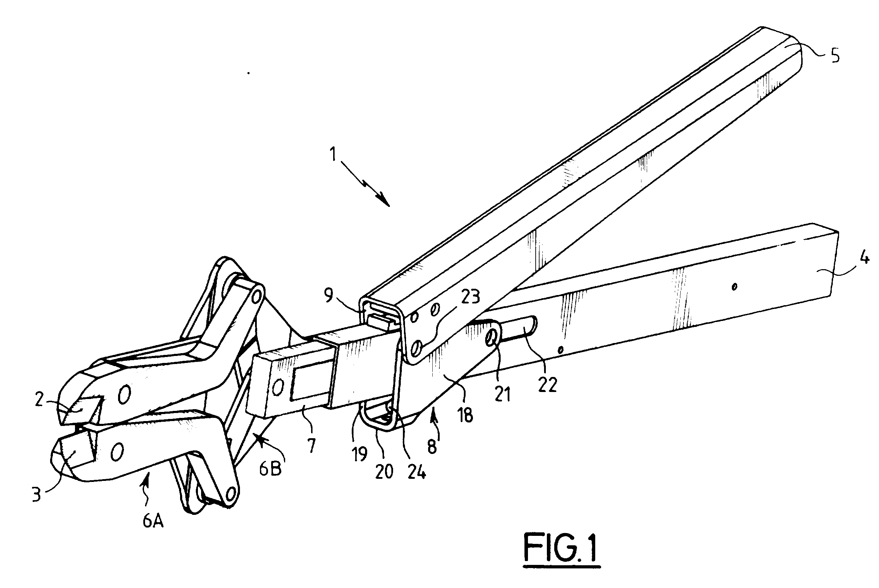

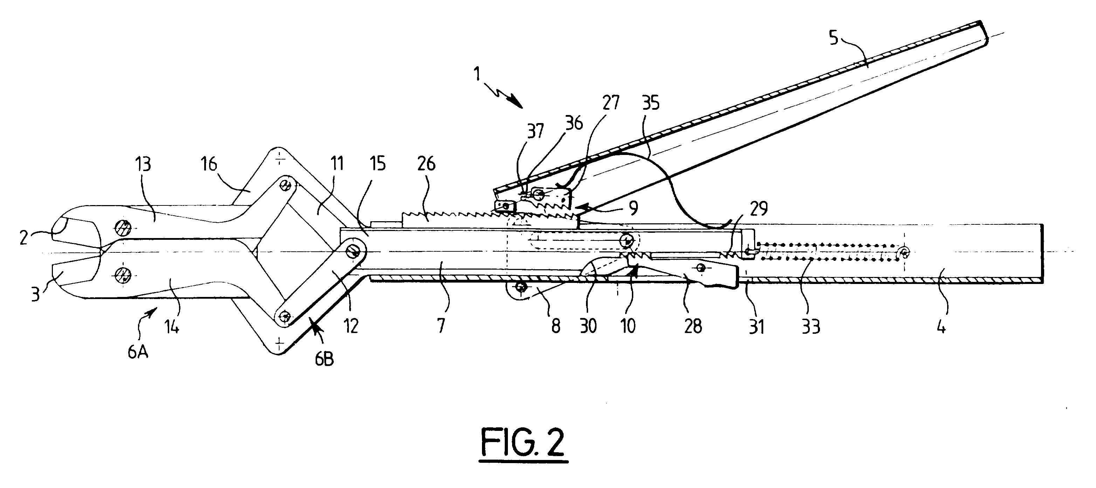

Le dispositif développeur d'efforts 1 représenté aux figures 1 et 2 est appliqué à une pince munie de deux becs 2 et 3 et de deux poignées, une première poignée 4 ou poignée fixe et une deuxième poignée 5 ou poignée mobile. La poignée mobile 5 est de section transversale en U et la poignée fixe 4 est creuse et de section transversale rectangulaire, la poignée 5 étant de longueur inférieure à celle de la poignée 4. Ces deux poignées 4 et 5 peuvent être réalisées soit en matière plastique, soit en matière métallique.

Le dispositif développeur d'efforts 1 comporte une mâchoire 6A et un système à biellettes 6B relié à un piston 7 logé dans la poignée fixe 4, des moyens 8 de maintien de la poignée mobile 5 par rapport à la poignée fixe 4, des moyens complémentaires 9 d'entraînement mécanique du piston 7 à l'intérieur de la poignée fixe 4 et des moyens anti-retour 10 du piston 7.

Le système à biellettes 6B est articulé sur la mâchoire 6A. Il est constitué de deux paires de biellettes 11 et 12 (pour la clarté des dessins, seule une biellette sur deux a été représentée pour chaque paire). Chaque paire de biellettes 11 et 12 comporte deux biellettes rectilignes parallèles. La mâchoire 6A possède deux biellettes 13 et 14 coudées, à leur extrémité avant, dans des sens opposés. L'ensemble des quatre biellettes 11 à 14 est agencé de manière à former sensiblement un quadrilatère.

Les extrémités arrière des paires de biellettes 11 et 12 sont chacune articulées sur l'extrémité avant 15 du piston 7 et de part et d'autre de celui-ci, tandis que les extrémités avant de ces biellettes sont respectivement articulées avec les extrémités arrière des biellettes 13 et 14. Les extrémités avant de ces biellettes 13 et 14 sont indépendamment articulées en deux points distincts placés sur un flasque 16 fixé sur la poignée fixe 4. Les extrémités avant de ces deux dernières biellettes portent respectivement les becs 2 et 3.

Le piston 7, de section transversale rectangulaire, est logé à coulissement dans la poignée fixe 4.

Les moyens 8 de maintien de la poignée mobile 5 par rapport à la poignée fixe 4 comportent un étrier 8 enfilé sur la poignée fixe 4. Cet étrier 8 comporte deux joues 18 et 19 de forme triangulaire qui sont reliées par un rebord plat 20. Les extrémités arrière des joues 18 et 19 sont solidaires du piston 7 par l'intermédiaire de tenons 21. Ces tenons 21 coulissent à l'intérieur de deux lumières longitudinales 22 ménagées dans chacune des faces latérales de la poignée fixe 4. L'extrémité avant de la poignée mobile 5 est articulée sur l'étrier 8 par l'intermédiaire de deux pivots 23 placés sur les extrémités avant des joues 18 et 19.

L'étrier 8 est en appui sur la face de la poignée fixe 4 qui est située à l'opposé de la poignée mobile 5. Cet appui est un appui glissant réalisé par l'intermédiaire d'un galet 24.

Les moyens complémentaires 9 d'entraînement mécaniques du piston 7 à l'intérieur de la poignée fixe 4 comportent une première crémaillère 26 portée fixement par la face de la poignée fixe 4 située en regard de la poignée mobile 5, et une deuxième crémaillère 27 portée à rotation par la face intérieure de la poignée mobile 5. La deuxième crémaillère rotative 27 est de longueur nettement inférieure à la première crémaillère 26 fixe et est placée en regard de celle-ci.

En variante, la deuxième crémaillère 27 peut être remplacée par un cliquet porté également intérieurement par la poignée 5 et à rotation ou par tout autre dispositif approprié.

Les moyens anti-retour 10 du piston 7 sont constitués par une gâchette 28 et une crémaillère 29. L'extrémité arrière de la gâchette 28 est portée à rotation par la face intérieure de la poignée fixe 4 de manière à ce que cette gâchette soit située en regard d'un évidement 30 ménagé dans la partie arrière du piston 7. L'extrémité avant de cette gâchette 28 porte une denture qui est en regard de la crémaillère 29 portée par l'évidement 30 et l'extrémité arrière s'étend hors de la poignée 4 au travers d'une ouverture 31.

L'extrémité arrière du piston 7 est en outre reliée à l'extrémité avant d'un ressort 33 dont l'extrémité arrière est portée par la face intérieure de l'extrémité arrière de la poignée fixe 4.

Des moyens élastiques du type ressort à lame 35 sont interposés entre la poignée fixe 4 et la poignée mobile 5. Ce ressort à lame a une forme générale en S dont l'extrémité arrière est fixée sur la face de la poignée fixe 4 en regard de la poignée mobile 5 et l'extrémité avant est en appui contre la face de la crémaillère 27 opposée à la denture de cette crémaillère, un sommet de ce ressort à lame 35 étant en appui contre la face intérieure de la poignée mobile 5.

L'extrémité avant de la crémaillère rotative 27 possède une saillie 36 destinée à venir en butée, sous l'action de la lame ressort 35, contre un ergot 37 placé dans l'une des faces latérales de la poignée mobile 5.

Bien que cela ne soit pas représenté aux figures, les becs 2 et 3 peuvent être des becs amovibles, du type becs de cisaillement ou becs de serrage.

Le fonctionnement de ce dispositif va maintenant être expliqué en regard des figures 2 à 5.

Au repos, c'est-à-dire lorsqu'on exerce aucune action sur cette pince, les deux poignées fixe 4 et mobile 5 sont sollicitées en écartement par la lame ressort 35. Le piston 7 est sollicité en arrière par le ressort 33 en ayant au préalable désenclenché la gâchette 28 de la crémaillère 29. Les becs 2 et 3 sont ainsi écartés l'un de l'autre. De même, les crémaillères 26 et 27 n'engrènent pas l'une avec l'autre et l'extrémité arrière de la crémaillère 26 est placée en regard de la crémaillère 27.

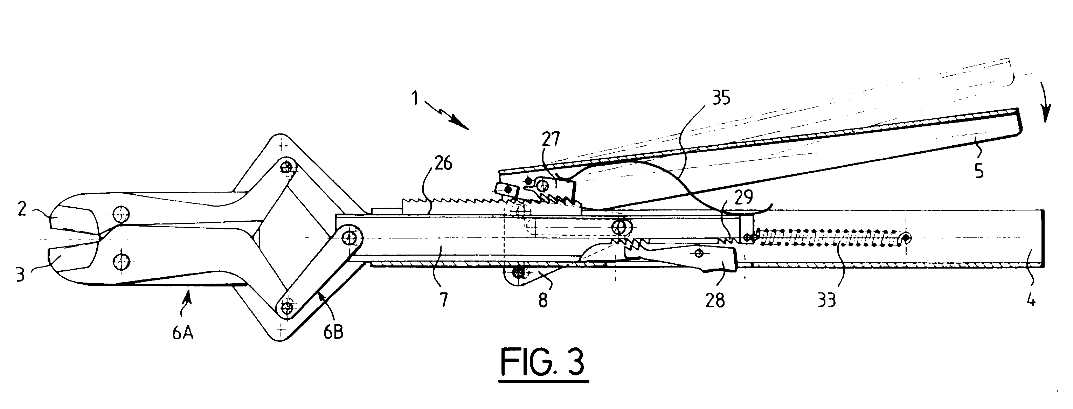

Lorsque l'on veut exercer une action de coupe ou de serrage par l'intermédiaire des becs 2 et 3, on exerce sur la poignée mobile 5 une action de manière à rapprocher les deux poignées 4 et 5 (figure 3). Les crémaillères 26 et 27 engrènent alors l'une avec l'autre, ce qui provoque un mouvement d'avance du piston 7 à l'intérieur de la poignée fixe 4. Ce mouvement d'avance a lieu jusqu'à ce que toutes les dents de la crémaillère 27 engrènent avec les dents de la crémaillère 26 et provoque une déformation du système à biellettes 6B vers l'avant ainsi qu'un écartement des extrémités arrière des deux biellettes 13, 14 de la mâchoire 6A par rotation. Cet écartement des deux biellettes 13 et 14 entraîne un premier rapprochement des becs 2 et 3 l'un vers l'autre. La denture de la gâchette 28 coopère alors avec la denture 29 placée à l'arrière du piston 7, ce qui immobilise ce piston en position.

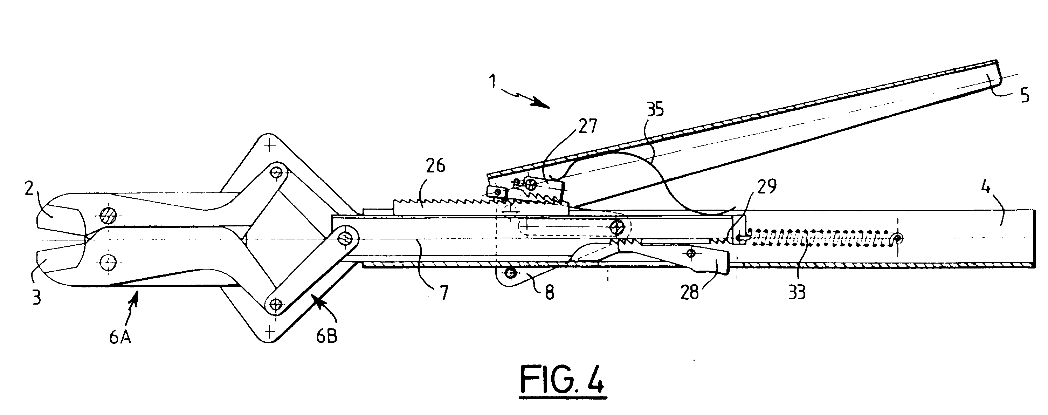

L'effort sur la poignée mobile 5 peut alors être relâché (figure 4), ce qui libère la crémaillère 27 de la crémaillère 26, tout en maintenant les becs 2 et 3 rapprochés.

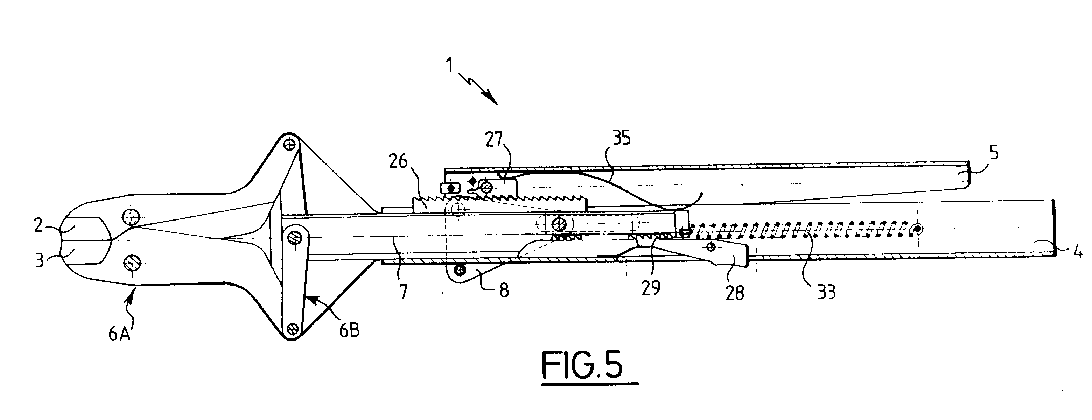

Par une action identique à la précédente, on rapproche à nouveau les deux poignées fixe 4 et mobile 5, le piston 7 est alors entraîné à nouveau vers l'avant. Ce mouvement d'avance resserre un peu plus les becs 2 et 3. Par mouvements successifs de rapprochement et d'éloignement de la poignée mobile 5 par rapport à la poignée fixe 4, c'est-à-dire par un mouvement analogue à un pompage, on rapproche progressivement les becs 2 et 3 l'un de l'autre jusqu'à obtenir un serrage ferme ou un effort de coupe très important (figure 5) et cela en huit ou neuf pompages successifs.

Lorsque l'on désire à nouveau écarter les becs 2 et 3 l'un de l'autre, il suffit de libérer la denture de la gâchette 28 par rapport à la denture de la crémaillère 29 du piston 7 en appuyant sur l'extrémité arrière de cette gâchette 28. Le ressort 33 rappelle alors le piston 7 vers l'arrière.

Ce dispositif développeur d'efforts d'une pince a pour avantage de développer des efforts de serrage ou de coupe très importants en exerçant une simple action de pompage sur les poignées de cette pince, à l'aide d'une seule main. Ce dispositif est donc très peu encombrant, permettant une intervention facile in situ. De plus, puisque l'effort nécessaire à la coupe totale est obtenu en plusieurs pompages, les becs subissent une contrainte considérablement réduite par rapport au cas où il est apporté en une seule manoeuvre, de sorte que les sections desdits becs peuvent être réduites d'autant, ce qui favorise leur faible encombrement.

## Revendications

1. Dispositif développeur d'efforts (1) d'une pince du type munie de deux becs (2, 3) et de deux poignées (4, 5), caractérisé en ce qu'il comporte :
- une mâchoire (6A) formée de deux biellettes (13, 14) coudées dans des sens opposés et articulées, au niveau de leur partie coudée, indépendamment l'une de l'autre, les deux becs (2, 3) étant positionnés à une de leurs extrémités ;
- un système à biellettes (6B) articulé avec les deux biellettes (13, 14) de la mâchoire (6A), l'ensemble mâchoire et système à biellettes ayant sensiblement la forme d'un quadrilatère déformable ;
- un piston (7) relié par son extrémité avant au système à biellettes (6B) au niveau d'un sommet de ce système opposé au sommet de la mâchoire (6A) portant les becs (2, 3), le piston (7) étant logé à l'intérieur d'une première poignée (4) et guidé à coulissement par celle-ci ;
- des moyens de maintien (8) de la seconde poignée (5) par rapport à la première poignée (4) solidaires du piston (7) et prenant appui sur la première poignée (4) ;
- des moyens complémentaires (9) d'entraînement mécanique du piston (7) à l'intérieur de la première poignée (4), portés par chacune des deux poignées (4, 5) ; et
- des moyens anti-retour (10) du piston (7) portés par la première poignée (4) ;
les moyens complémentaires (9) d'entraînement du piston (7) et les moyens anti-retour (10) agissant alternativement sur celui-ci de manière à provoquer, par mouvements successifs de rapprochement et d'éloignement de la seconde poignée (5) par rapport à la première poignée (4), un déplacement du piston (7) vers l'avant et à déformer la mâchoire (6A) par l'intermédiaire du système à biellettes (6B) pour rapprocher les deux becs (2, 3) l'un vers l'autre.

2. Dispositif selon la revendication 1, caractérisé en ce que les moyens d'entraînement mécanique (9) comprennent une première crémaillère (26) portée fixement par la première poignée (4) et une deuxième crémaillère (27), de longueur inférieure à la première crémaillère (26), articulée sur la deuxième poignée (5) et placée en regard de la première crémaillère (26), de manière à s'engréner mutuellement lors du rapprochement des deux poignées (4, 5).

3. Dispositif selon la revendication 1, caractérisé en ce que les moyens d'entraînement mécanique (9) comprennent une crémaillère (26) portée fixement par la première poignée (4) et un cliquet porté par la deuxième poignée (5) de manière à coopérer avec ladite crémaillère (26) lors du rapprochement des deux poignées (4, 5).

4. Dispositif selon la revendication 2 ou 3, caractérisé en ce qu'il comporte des moyens élastiques (35) portés par les deux poignées (4, 5), de façon à solliciter ces poignées en éloignement l'une de l'autre et à appliquer la deuxième crémaillère (27) ou le cliquet sur la première crémaillère (26) lors du rapprochement des poignées (4, 5).

5. Dispositif selon l'une quelconque des revendications 1 à 4, caractérisé en ce que le système à biellettes (6B) comprend deux paires (11, 12) de biellettes, chaque paire étant articulée à l'extrémité avant du piston (7) et à l'une des deux biellettes (13, 14) de la mâchoire (6A).

6. Dispositif selon l'une quelconque des revendications 1 à 5, caractérisé en ce que les moyens anti-retour (10) du piston (7) comprennent une gâchette (28) portée par la première poignée (4) et coopérant avec une crémaillère supplémentaire (29) placée sur le piston (7).

7. Dispositif selon l'une quelconque des revendications 1 à 6, caractérisé en ce que les moyens de maintien (8) de la seconde poignée (5) par rapport à la première poignée (4) comprennent un étrier (8) orienté à l'opposé de la première crémaillère (26) et dont les deux joues (18, 19) sont parallèles et disposées de part et d'autre de la première poignée (4).

8. Dispositif selon la revendication 7, caractérisé en ce que les extrémités arrière des joues (18, 19) sont solidaires du piston (7) et la deuxième poignée (5) est articulée par deux pivots (23) placés à l'avant des joues (18, 19).

9. Dispositif selon la revendication 7 ou 8, caractérisé en ce que l'étrier (8) est en appui sur la première poignée (4) par l'intermédiaire d'un galet (24) sur la face de ladite première poignée (4) opposée à celle portant la première crémaillère (26).

10. Dispositif selon l'une quelconque des revendications 1 à 9, caractérisé en ce que les moyens anti-retour (10) du piston (7) sont désenclenchables.

11. Dispositif selon la revendication 10, caractérisé en ce qu'il comporte des moyens élastiques (33) sollicitant le piston (7) vers l'arrière lorsque les moyens anti-retour (10) sont désenclenchés.

12. Dispositif selon l'une quelconque des revendications 1 à 11, caractérisé en ce que les becs (2, 3) sont des becs amovibles.

13. Pince coupante comprenant le dispositif défini selon l'une quelconque des revendications 1 à 12, et dans laquelle les becs (2, 3) sont des becs de cisaillement.

14. Pince de serrage comprenant le dispositif défini selon l'une quelconque des revendications 1 à 12, et dans laquelle les becs (2, 3) sont des becs de serrage.
